Europäisches Patentamt

European Patent Office

Office européen des brevets

(19)

(11) **EP 1 428 528 A1**

(12) **EUROPEAN PATENT APPLICATION**

(43) Date of publication:
 **16.06.2004 Bulletin 2004/25**

(51) Int Cl.⁷: **A61K 31/19**, A61K 31/715,
 A61P 35/00
 // (A61K31/19, 31:715)

(21) Application number: **02021644.6**

(22) Date of filing: **27.09.2002**

| | |
|---|---|
| (84) Designated Contracting States:<br>**AT BE BG CH CY CZ DE DK EE ES FI FR GB GR**<br>**IE IT LI LU MC NL PT SE SK TR**<br>Designated Extension States:<br>**AL LT LV MK RO SI**<br><br>(71) Applicant: **Tiense Suikerraffinaderij N.V.**<br>**1150 Brussel (BE)**<br><br>(72) Inventors:<br>• **Van Loo, Jan**<br> **3040 St. Agatha Rode (BE)** | • **Frippiat, Anne**<br> **1933 Sterrebeek (BE)**<br>• **Hermans, Johny**<br> **3150 Haacht (BE)**<br><br>(74) Representative: **Hermans, Johny**<br> **Tiense Suikerraffinaderij N.V.,**<br> **Patent Department,**<br> **Aandorenstraat 1**<br> **3300 Tienen (BE)** |

(54) **Synergistic combinations of dietary fiber and NSAIDs for the treatment of cancer**

(57)    Pharmaceutical compositions are disclosed that present synergistic anti-cancer effects in humans and vertebrate animals, comprising a combination of a fermentable dietary fiber (FDF) and a non-steroidal anti-inflammatory drug (NSAID) with cyclo-oxygenase2 (COX2) inhibiting activity, the FDF being present in the FDF/ NSAID combination in a weight ratio FDF : NSAID of minimum 1:1. Typical compositions comprise a FDF/ NSAID combination wherein the FDF component is chicory inulin, oligofructose or a mixture thereof, and the NSAID component is selected from a group consisting of sulindac, piroxicam or aspirin, in a weight ratio FDF : NSAID ranging from 6000 : 1 to 1:1. The synergistic anti-cancer effects of the compositions are demonstrated in aberrant crypt foci studies in rats.

The pharmaceutical compositions are useful in the prevention and inhibition of carcinogenesis or recurrence of cancer and for the treatment of cancer in humans and vertebrate animals.

The invention also relates to a method for prevention or inhibition of carcinogenesis and recurrence of cancer and for the treatment of cancer in humans and vertebrate animals involving administration of a therapeutically effective dose of said pharmaceutical compositions to a human or vertebrate animal.

**EP 1 428 528 A1**

**Description**

Field of the invention

The invention relates to a pharmaceutical composition with synergistic anti-cancer properties comprising a combination of a fermentable dietary fiber and a non-steroidal anti-inflammatory drug, to a method for preparing same, to a method for the prevention and inhibition of carcinogenesis and the recurrence of cancer, and to a method for the treatment of cancer.

Background and prior art

In industrialised countries, cancer has become an important cause of death of humans. Although cancer appears in various kinds, it is generally known to be a disease involving a complex, multi-step process. The origin and development of cancer, also termed herein carcinogenesis, is generally considered to proceed in three major steps, namely initiation, promotion and progression. The initiation step is characterised by a modification of the genome of normal cells that imparts selective growth advantage to the modified cells. The modification is caused by exposure of normal cells to carcinogens of chemical, physical or viral nature. In the promotion step, the initiated, modified cells express their altered genome by a clonal proliferation which is generally associated with the formation of detectable, morphologically and/or phenotypically changed tissue structures, such as premalignant foci, benign nodules and benign neoplasms. In the progression step, the benign neoplastic cells are transformed into malignant tumour cells that proliferate in an uncontrolled manner, locally invading adjacent normal body tissues and organs and finally spreading into distant body tissues and organs by the metastases. The invasion of normal body structures results in their malfunctioning and destruction, which eventually leads to the death of the affected human. Cancer is also affecting many vertebrate animals in which the disease generally occurs through a mechanism similar to the one observed in humans.

Various methods for the treatment of cancer have already been developed including treatment by surgery, irradiation and chemotherapy, and by combinations thereof. The method of treatment is largely defined having regard to the kind, the location and the stage of development of the cancer, and the physical condition of the affected human.

On the one hand, technological developments make invasive techniques, including surgery and irradiation, more and more efficient. However, these treatments require highly sophisticated equipment and cause major discomfort to the patient, making them costly and feared by the patients.

On the other hand, great research efforts resulted in the discovery of many compounds with anti-cancer activity, some of which are proven suitable as anti-cancer drugs for the treatment of cancer by chemotherapy. A major advantage of anti-cancer drugs resides in the fact that they are easy to administer and often enable non-invasive treatment of cancer. Accordingly, chemotherapy with anti-cancer drugs, in combination or not with other treatment methods, has become an important way of treatment of cancer.

However, many anti-cancer drugs present serious drawbacks too. Indeed, several drugs present a high degree of toxicity for cells of normal body structures causing, for example, liver and kidney damages, or local necrosis of the body structure in which the drug is parenterally administered. Anti-cancer drugs may also cause an increased sensitivity to opportunistic infections and provoke various types of discomfort for the treated person, such as, nausea, vomiting, irritation of the mucosa of the digestive tract and/ or diarrhoea when the drug is administered parenterally, orally or via tube feeding. Accordingly, drawbacks may considerably limit the use of anti-cancer drugs. One may, for example, not be able to give to a patient a curative effective dose of an anti-cancer drug because of the too high toxicity of the drug to normal cells or the too high degree of discomfort caused by the drug to the patient.

Anti-cancer drugs are conventionally classified in one of the following groups:

    1. alkylating agents,
    2. anti-metabolic drugs,
    3. anti-mitotic antibiotics,
    4. alcaloidal anti-tumour agents,
    5. hormones and anti-hormones,
    6. interferons,
    7. non-steroidal anti-inflammatory drugs, and
    8. various other anti-tumour agents.

Non-steroidal anti-inflammatory drugs (generally abbreviated as NSAID('s)), form a recent class of anti-cancer agents since it was discovered that administration of NSAID's reduces carcinogenesis and recurrence of cancer. For example, administration of sulindac to patients with familial adenomatous polyposis was found to significantly reduce the number and size of colorectal adenomas compared to patients given placebo (Giardiello (1993)). Accordingly, NSAID's, that were commonly only used as analgesic and anti-inflammatory drugs, find more and more application in the treatment of cancer after treatment by surgery to reduce the risk of recurrence and to inhibit the recurrence of cancer.

NSAID's were found to inhibit prostaglandin synthesis by blocking the activity of cyclo-oxygenase (COX), an enzyme that has two isoforms, namely cyclo-oxygenasel (herein COX1) and cyclo-oxygenase2 (herein COX2).

Prostaglandins produced under the influence of the isoenzyme COX1 play an important role in maintaining the integrity of the gastroduodenal mucosa, mediate normal platelet function and regulate renal blood flow. Prostaglandins produced under the influence of the isoenzyme COX2 are primarily associated with inflammation.

Traditional NSAID's are non-selective COX inhibitors that inhibit prostaglandin synthesis through inhibition of both COX1 and COX2 isoforms. Blockage of COX1 causes the known undesirable gastrointestinal side effects associated with prolonged intake of non-selective COX inhibitors.

Discovery of the COX isoforms and their function led to the development of selective NSAID's which present selective COX2 inhibition activity while maintaining the pharmacological potential of NSAID's without causing the undesirable COX1 associated gastro-intestinal side effects. Typical examples of NSAID's that are non-selective COX inhibitors are aspirin and piroxicam. A typical example of a NSAID that is a selective COX2 inhibitor is sulindac.

It has been observed that COX2 activity is upregulated in colon cancer cells and that said activity can be normalized by NSAID's due to their inhibiting effect on COX2 activity. On the basis thereof it is now assumed that, as COX2 inhibitors, NSAID's exert inhibitory effects on carcinogenesis and are able to reduce the risk for cancer in healthy persons.

Furthermore, certain dietary components have been identified which interact with physiological functions in the body in a manner to improve them or to restore them more or less in case of a dysfunction. These components are conventionally termed functional food components, a particular class of which is formed by the so-called dietary fibers. Dietary fibres are non-digestible carbohydrates, namely carbohydrates that are resistant to hydrolysis by the digestive enzymes of humans. Dietary fibres mostly occur as components of plant cells. Dietary fibers include, for example, cellulose, hemi-cellulose, lignin, pectin, gums, waxes, resistant starch, fructan, and certain other non-digestible carbohydrates. Depending on the chemical structure, dietary fibers may be water soluble or not, and may or may not be metabolised by fermentation by intestinal bacteria that are present in the large intestine, in particular bacteria of the genus *Bifidus* and *Lactobacillus*. Accordingly, dietary fibers are commonly classified in non-fermentable dietary fibers, such as lignin, cellulose and hemicellulose, and in fermentable dietary fibers, such as resistant starch and partially hydrolysed resistant starch, fructan and partially hydrolysed fructan, pectin and partially hydrolysed pectin, galactomannans and partially hydrolysed galacto-mannans, beta-glucans, gums, for example arabic gum and guar gum, partially hydrolysed gums, such as partially hydrolysed guar gum (for example Benefiber®, trade name of Novartis Nutrition), and fermentable dietary fibers of the class of oligosaccharides, such as for example fructo-oligosaccharides, galacto-oligosaccharides, xylo-oligosaccharides, gluco-oligosaccharides, soybean oligosaccharides and arabinogalactans. Dietary fibers, in particular fermentable dietary fibers, are well known in the art.

Fermentable dietary fibers (in short herein FDF) were found to present preventive effects on the incidence of cancer, and inhibitory effects on carcinogenesis and possibly on the development of cancer (Howe et al., (1992)).

EP 0 692 252 A1 discloses a composition containing a fructan, particularly oligofructose or inulin, that presents preventive and inhibitory effects on carcinogenesis and on the growth of mammary cancer in mammals. EP 0 692 252 A1 also discloses in a generic manner that said compositions may additionally comprise conventional chemotherapeutic products that actively destroy malignant tumour cells. In Example 7 of EP 0 692 252 A1 is mentioned that to determine potential synergistic therapeutic effects, a pharmaceutical composition comprising RAFTILINE® (trade name for chicory inulin of Raffinerie Tirlemontoise s.a. /Tiense Suikerraffinaderij n.v., Belgium [in short herein "RT/TS, Belgium"]) and a conventional chemotherapeutic product actively destroying malignant tumour cells, is prepared and a test is described wherein doxorubicine (an anti-cancer drug of the class of antimitotic antibiotics) was injected into mice fed RAFTILINE® which were previously inoculated with L1210 leukaemic tumour cells. However, EP 0 692 252 A1 is completely silent about the outcome of the test and about possible synergistic anti-cancer effects between inulin and conventional anti-cancer drugs.

WO 98/52578 discloses the use of fructan-type carbohydrates, particularly long-chain chicory inulin, for the manufacture of a composition for oral administration or tube feeding for the prevention and treatment of colon cancer in humans and non-bovine mammals. WO 98/52578 also discloses in a generic manner that the composition may optionally comprise a physiologically active substance, a drug or pro-drug.

WO 01/60176 discloses in example 5 the anti-cancer effects of a particular inulin product on azoxymethane-induced carcinogenesis in rat colon. The inulin product is a particular combination of easily fermentable inulin (short-chain inulin, typically oligofructose) and hardly fermentable inulin (typically long-chain chicory inulin).

Furthermore, the combined effect of levan and four cytotoxic/anti-cancer agents on the growth of experimental tumours in mice was published by Leibovici et al. (1983). The cytotoxic/anti-cancer agents were cyclophosphamide (class of alkylating agents, methotrexate (class of anti-metabolite agents), vincristine (class of alcaloidal anti-tumour agents), and 5-fluoro-uracil (class of anti-metabolite agents). Only additive effects were observed with all the combinations of levan and the cytotoxic agents, except for the combination of levan and methotrexate, an anti-metabolite anti-cancer agent, which gave a synergistic effect on Lewis lung carcinoma. However, no synergistic anti-cancer effect was observed for the combination of levan and 5-fluoro-uracil, also an anti-metabolite anti-cancer drug.

WO 99/59600 discloses synergistic anti-cancer effects of a pharmaceutical composition comprising a combination of inulin, oligofructose or a mixture thereof and an anti-metabolic anticancer drug. WO 99/59600 furthermore discloses (pages 12 to 14) comparative tests wherein the anti-cancer effects of compositions comprising inulin or oligofructose and an anti-cancer drug from different classes of anti-cancer drugs are examined on mice in which viable, neoplastic cells from a transplantable mouse liver tumour were intraperitoneally transplanted. The examined anti-cancer drugs were endoxan (class of alkylating agents), adriamycin (class of anti-mitotic antibiotics), 5-fluoro-uracil (class of anti-metabolite agents) and oncovin (class of alcaloidal anti-tumour agents). For the treatment with the composition comprising oncovicin, endoxan and adriamycin the therapeutic effects observed were only additive, not synergistic. Only for compositions with 5-fluoro-uracil synergistic anti-cancer effects were observed, and no significant difference in effects between compositions comprising inulin or oligofructose was noted.

The prior art data thus clearly showed that only very specific combinations of dietary fibers and anti-cancer agents, namely combinations of fructan and anti-metabolite anti-cancer agents, present synergistic anti-cancer effects, whereas the combinations of fructan and compounds of other classes of anti-cancer agents merely present additional anti-cancer effects.

In view of the social and economical impact of cancer, great efforts are continuously made to find new or improved products, compositions and methods for reducing the incidence of cancer, for the prevention or inhibition of carcinogenesis and for the treatment of cancer, and for reducing the noxious effects of cancer. In particular, there is an on-going search to find highly effective anti-cancer products and pharmaceutical compositions, that present good therapeutic anti-cancer effects, preferably with a high degree of specificity and a low degree of toxicity to normal cells, and that provoke a minimum of discomfort to the patient.

Description of the invention

The present invention is based on the findings of the inventors that a combination of a fermentable dietary fiber and a non-steroidal anti-inflammatory drug (both, selective COX2 inhibitors and non-selective COX inhibitors), not only presents additive anti-cancer effects but surprisingly presents synergistic anti-cancer effects.

The invention, presenting a solution to one or more of the above problems, relates in a first aspect, to a pharmaceutical composition that presents synergistic anti-cancer effects and that is suitable for the prevention and inhibition of carcinogenesis and the recurrence of cancer, and for the treatment of cancer in humans and vertebrate animals, in particular fish, birds and mammals, particularly non-ruminating mammals, typically pets, such as dogs and cats.

The pharmaceutical composition according to the invention is characterised in that it comprises a combination of an effective amount of a fermentable dietary fiber (FDF) and an effective amount of a non-steroidal anti-inflammatory drug (NSAID) with cyclo-oxygenase2 (COX2) inhibiting activity, namely an anti-cancer drug of the class of non-steroidal anti-inflammatory drugs (NSAID's), the FDF component being present in the combination in a weight ratio FDF/NSAID of minimum 1 : 1, typically a weight ratio ranging from about 6000 :1 to 1 : 1, preferably from 2000 : 1 to 5 : 1, more preferably from 750 : 1 to 10 : 1, most preferably from 500 : 1 to 10 : 1. The presence of the FDF and NSAID in the said weight ratio results in synergistic anti-cancer effects.

By combination is meant herein a mixture or association of the FDF and the NSAID. The mixture or association can be prepared by conventional techniques. The mixture can, for example, be obtained by mere dry mixing of the FDF and NSAID in a predetermined weight ratio, and the association can, for example, be obtained by mixing of the components FDF and NSAID in solution, suspension or emulsion, optionally followed by co-drying of the resulting combination by conventional techniques, for example co-spray drying. The combination can also be prepared by coating the FDF in particulate form partially or completely with the NSAID by conventional techniques.

The combination can be used as such, thus forming a composition according to the invention, for example in the form of a powder, granulate or tablet, for the prevention or inhibition of carcinogenesis or recurrence of cancer and for the treatment of cancer. However, usually the combination will be used in the form of a pharmaceutical composition brought by conventional techniques in an adequate galenic form that is suitable for the intended way of administration.

The pharmaceutical composition of the invention can be prepared by conventional techniques, for example by mixing a said, previously prepared, combination of the FDF and the NSAID according to the invention, with the other pharmaceutically acceptable components, or by mixing all the components of the pharmaceutical composition including the desired amounts of the active components FDF and NSAID, optionally followed, if appropriate, by co-drying. The components of the composition other than the FDF and NSAID are conventional, pharmaceutically acceptable ingredients, for example one or more solvents, diluents, excipients, and/or additives used in galenic pharmacy. Optionally, the composition may comprise further physiologically active components and drugs.

By the term cancer is meant herein any kind of cancer occurring in humans and in vertebrate animals, irrespective of the stage of development of said cancer, thus including carcinogenesis, with in particular, the initiation, promotion, and progression stage, the metastasis stage, as well as the recurrence of cancer after treatment of a cancer.

By the terms anti-cancer effect(s), therapeutic anti-cancer effect(s) and anti-cancer properties (used interchangeably herein) is meant herein prophylactic effects (namely effects that prevent carcinogenesis and the recurrence of cancer), as well as therapeutic effects (namely inhibitory effects on carcinogenesis, on the development of cancer, on the progression of the cancer disease and on recurrence of cancer, as well as curative effects provoking the reduction of the number and/ or size of neoplasms, and/ or the regression or curing of the cancer disease).

By treatment of cancer is meant herein the therapeutic treatment of cancer in all its stages.

By effective amount of FDF and effective amount of NSAID is meant herein an amount of each of said components that in combination with each other presents synergistic anti-cancer effects in a human or animal, namely increased anti-cancer effects compared to the sum of the anti-cancer effects presented by each of the components taken separately.

By non-steroidal anti-inflammatory drug (NSAID) is meant a drug that has an inhibitory effect on cyclo-oxygenase2 (COX2) enzyme activity. The term NSAID herein embraces non-selective NSAID's, presenting both COX1 and COX2 inhibiting activity, as well as selective NSAID's, presenting only COX2 inhibiting activity.

It is understood that the term anti-cancer drug or anti-cancer agent (used interchangeably) herein includes, in particular with respect to NSAID's, the drug *per se* as well as a pro-drug, namely a compound which is transformed in the body into the corresponding drug.

Fermentable dietary fibers have already been defined above and each of them is suitable as FDF component in the FDF-NSAID combination of the composition of the present invention. Very suitable fermentable dietary fibers include compounds of the class of fructan(s), namely carbohydrates that are classified in levan-type fructan and in inulin-type fructan, both well known in the art. Levan is composed of polyfructose molecules wherein the fructose units are mostly connected to each other by $\beta$(2-6) fructosyl-fructose linkages. Inulin is composed of polyfructose molecules that mainly consist of fructose units that are connected to each other mostly or exclusively by $\beta$(2-1) fructosyl-fructose linkages. In inulin molecules the polyfructose chain often ends in a glucose unit. Because $\beta$(2-6) and $\beta$(2-1) fructosyl-fructose linkages are not hydrolysed by digestive enzymes of humans and most animals, levan and inulin pass almost unaltered through the digestive tract into the large intestine (colon) where they are fermented by intestinal bacteria.

Inulin molecules are commonly represented by the general formulae $GF_n$ and $F_m$ wherein G represents a glucosyl unit, F represents a fructosyl unit, and n and m represent the number of fructosyl units in the carbohydrate chain. The number of saccharide units (fructose and glucose units) in one inulin molecule, *i.e.* the values n+1 in formula GFn and m in formula Fm, are referred to as the degree of polymerisation, represented by DP. Often the parameter "average degree of polymerisation", represented by av. DP or $(\overline{DP})$, is used too, which is the value corresponding to the total number of saccharide units divided by the total number of inulin molecules present in a given inulin sample, without taking into account present mono- and disaccharides (De Leenheer, 1996).

Inulin, which is well known in the art, is synthesised by many plant species, can originate from bacterial activity and can be enzymatically synthesised for example from sucrose. Depending on its origin, inulin occurs as a polydisperse mixture of linear and/ or branched polyfructose molecules. Inulin from plant origin is a polydisperse mixture of polyfructose molecules with a DP ranging from 3 to about 100, whereas inulin from bacterial origin usually has a higher DP. Enzymatically synthesised inulin mostly has a DP lower than 10.

At industrial scale, inulin is mainly obtained from roots of chicory and is commercially available in various grades, for example as RAFTILINE® (trade name of RT/ TS, Belgium). Typical RAFTILINE® grades include ST (which has an av. DP of at least 10, typically of 10 to 13, and contains in total about 8 wt % glucose, fructose and sucrose), LS (which has an av. DP of at least 10, typically of 10 to 13, but contains in total less than 1 wt% glucose, fructose and sucrose), HP (which has an av. DP of at least 23 to about 30, typically of 23 to 25, and which is essentially free of glucose, fructose and sucrose).

Inulin molecules that have a DP ranging between 2 and 10 are conventionally named interchangeably oligofructose, inulo-oligosaccharide and fructo-oligosaccharide. Oligofructose can be obtained at industrial scale by partial, acidic or enzymatic hydrolysis of inulin according to well-known techniques. Several grades of oligofructose are commercially available, for example from ORAFTI (Belgium), under the trade name RAFTILOSE® (trade name of RT/TS, Belgium), such as RAFTILOSE® P95 which contains about 95 % by weight oligofructose with a DP ranging from 2 to 9 and which contains about 5 % by weight in total of glucose, fructose and sucrose.

Other suitable grades of inulin include mixtures of an easily fermentable inulin (EFI) and a hardly fermentable inulin (HFI) in a weight ratio EFI/HFI ranging from 10/90 to 70/30, with a total content of inulin with a DP 9 and DP10 of maximally 5 % by weight, which are described for example in WO 01/60176. A typical example of such EFI/HFI inulin mixture with an EFI/HFI weight ratio of 1/1, available from ORAFTI (Belgium), is RAFTILOSE®Synergy1 (trade name of RT/TS, Belgium).

In a preferred embodiment, the composition of the invention comprises a FDF/NSAID combination wherein the fermentable dietary fiber component is chicory inulin with an av. DP of 10 to 13. In another preferred embodiment, the FDF component is chicory inulin with an av. DP of 23 to about 30, more preferably 23 to 25. In still another preferred embodiment, the FDF component is oligofructose with a degree of polymerisation (DP) of 2 to 9. In still a further preferred embodiment the FDF component is an inulin mixture of EFI and HFI in a weight ratio EFI/HFI ranging from 10/90 to 70/30 with a total content of inulin with a DP 9 and DP 10 of maximally 5 % by weight, more preferably a mixture of oligofructose with a DP of 2 to 9 and a chicory inulin with an av. DP of 23 to 25 with a total content of inulin with a DP 9 and DP10 of maximally 5 % by weight, in a weight ratio EFI/HFI of 1/1 (in short herein Synergy1) or in a weight ratio EFI/HFI of 1/2 (in short herein Synergy2).

In a preferred embodiment of the composition according to the invention, the NSAID component of the FDF/ NSAID combination is selected from the group consisting of sulindac, sulindac sulfide, sulindac sulfoxide, sulindac sulfone, aspirin, piroxicam, ketyoprofen, diclofenac, ibuprofen, indomethacin, celecoxib, nimesulide, omeprazole, and mofarotene, more preferably the NSAID is sulindac, sulindac sulfide, sulindac sulfoxide, sulindac sulfone, or aspirin, most preferably sulindac.

For the prevention and inhibition of cancer or the recurrence of cancer and for the treatment of cancer, the composition according to the invention is administered in a therapeutically effective amount, namely an amount that generates anti-cancer effects in the treated human or animal.

The effective amount of FDF and NSAID in the pharmaceutical composition of the invention depends on various factors including in particular the kind of fermentable dietary fiber and of NSAID and the method of administration of the pharmaceutical composition. The therapeutically effective amount of the composition of the invention, the optimal galenic form and the way of administration of the composition, depend on various factors including the kind and stage of development of the cancer and the kind and physical condition of the affected being. The effective amounts of FDF and NSAID and the therapeutically effective amount of the composition of the invention can be determined by the skilled person through conventional methods.

The pharmaceutical composition of the invention can be formulated in the form of a single dose unit comprising a therapeutically effective amount of the composition, or in several partial, but still therapeutically effective, dose units the administration of which can be spread over a period of time. Usually the composition will be administered spread over several partial dose units that present anti-cancer effects, and preferably spread, possibly with certain intervals, over one or more days or weeks.

The pharmaceutical composition is formulated and administered in such a manner that a daily dose provides an effective amount of FDF, commonly ranging from about 1 g/ day to about 30 g/ day, typically from about 1 g to 15 g/ day, and an effective amount of the NSAID, commonly ranging from about 5 mg to about 10 g, typically from about 50 mg to about 5 g.

The pharmaceutical composition may also comprise, instead of one FDF and one NSAID, two or more FDF's and/ or two or more NSAID's.

The pharmaceutical compositions according to the invention are typically present in a conventional galenic form that is suitable for oral or for tube feeding. The compositions for oral administration can, for example, be liquids, gels or solids and appear, for example, as tablets, coated tablets, coated pills, capsules, granules, solutions, syrups, suspensions or emulsions. The compositions for administration by tube feeding can be solutions, syrups, suspensions or emulsions that optionally can be mixed with food compositions for tube feeding. Suitable galenic forms also include retard release forms as well as sustained release forms that are well known in the art.

A particularly interesting feature of the pharmaceutical compositions according to the invention resides in the fact that the active components of the FDF/NSAID combination, namely the FDF and the NSAID, can be administered to the human or animal

(i) either simultaneously and present in the same galenic formulation (that constitutes the pharmaceutical composition according to the invention),
(ii) or simultaneously but present in two separate galenic formulations (that constitute together the pharmaceutical composition according to the invention) and optionally via the same or via two different methods of administration,
(iii) or non-simultaneously, via two separate galenic formulations (that constitute together the pharmaceutical composition according to the invention) and optionally via the same or via two different methods of administration.

According to said methods of administration, the FDF as well as the NSAID, can be administered orally or via tube feeding. Furthermore, in said methods of administration wherein the FDF component and the NSAID component are administered either simultaneously or non-simultaneously but in separate galenic formulations, the NSAID may also be administered parenterally, either locally or systemically. The NSAID component has, of course, to be present in a galenic formulation that is suitable for the chosen method of administration. The formulation for parenteral administration can for example be a solution, an emulsion or a suspension.

When the FDF component and the NSAID component of the FDF/NSAID combination of a pharmaceutical composition according to the invention are not simultaneously administered to a being, it is compulsory that they are brought into the body of said being in such a manner that they are present in the body at least partly together, as a result of which the components can interact with each other to yield the synergistic anti-cancer effects in accordance with the present invention.

According to a preferred embodiment, both active components, FDF and NSAID, are administered simultaneously in one pharmaceutical composition to the human or animal. In another preferred embodiment, both active components are administered simultaneously but in separate formulations to the human or animal, optionally via a different method of administration. In a further preferred embodiment both active components are non-simultaneously administered in separate formulations and optionally via different methods of administration.

The FDF component can be administered in various galenic formulations, for example tablets, capsules, syrups, solutions, suspensions or emulsions. The FDF component can even be administered orally or via tube feeding in the form of a functional food or feed, i.e. a food or feed product which has been supplemented with the desired amount of FDF component. Typical examples of functional food or feed include dairy products (e.g. milk and yoghurts), jams, marmalades, baked goods (including bread and breakfast cereals), cereal bars, desserts (e.g. puddings), spreads, drinks, and meal replacers.

Furthermore, it may be beneficial to administer the same FDF as used in the composition of the invention to the human or animal already some time, for example one week, before the composition of the invention is administered. Without wishing to be bound by any theory, it is supposed that as a result of the prebiotic effect of the FDF administered prior to the composition of the invention, the intestinal flora and/ or certain bodily functions are brought into a condition in which the synergistic anti-cancer effects generated by the combination of the active components FDF and NSAID in the composition according to the invention can develop optimally.

In a further aspect, the present invention relates to the use of the said combination consisting of a FDF and a NSAID, for the manufacture of a pharmaceutical composition according to the present invention that presents synergistic anti-cancer effects.

In still a further aspect, the invention relates to a method for the prevention or inhibition of carcinogenesis and recurrence of cancer and for the treatment of cancer in humans and in vertebrate animals, comprising administering to said being in need for such treatment an effective dose of the pharmaceutical composition of the invention, comprising a combination consisting of a FDF and a NSAID as defined above, said active components FDF and NSAID being administered either simultaneously in the same or in separate formulations, or non-simultaneously in separate formulations.

The invention is illustrated by the following examples.

Example 1

In example 1 data are shown of comparative experiments wherein has been investigated whether dietary treatment with a fermentable dietary fiber is able to potentiate the therapeutic anti-cancer effects of NSAID's. By means of an aberrant crypt foci (ACF) study in rats, the anti-cancer effects were examined of a composition according to the present invention containing inulin or oligofructose and inulin as the FDF component and a selective cyclo-oxygenase2 inhibiting NSAID or a non-selective cyclo-oxygenase inhibiting NSAID as the NSAID component.

Experimental details

The inulin used was Synergy1 (in short Syn1) and Synergy2 (in short Syn2), which are mixtures of oligofructose (RAFTILOSE®) and inulin of av. DP of 23 to 25 (RAFTILINE®) with a total content of inulin with a DP 9 and DP 10 of maximally 5 % by weight, at a weight ratio of 1:1 and 1:2, respectively, obtained from ORAFTI (Belgium) (RAFTILOSE® and RAFTILINE® are trade names of RT/TS, Belgium). The diets were prepared fresh every week and stored at 4°C until fed. The experimental diets were formulated based on AIN 93G diets. The ingredients were obtained from ICN Biomedicals, Aurora, OH, USA. All other chemicals were obtained from Sigma Chemical Co., St. Louis, MO, USA. The composition of the diets is shown in Table 1 below. All the protocols involving rats have been approved by the Institutional Animal Care and Use Committee of Alabama A& M University.

Table 1:

| Composition of the diets * | | | | | | | | | | |
|---|---|---|---|---|---|---|---|---|---|---|
| Ingredient (in g/kg) | Contr | Syn1 | Syn2 | Sul | Piro | Aspi | Sul + Syn1 | Sul + Syn2 | Aspi + Syn1 | Piro + Syn1 |
| Corn Starch | 397.5 | 297.5 | 297.5 | 397.28 | 397.28 | 397.28 | 297.28 | 297.28 | 297.28 | 297.28 |
| Inulin | 0 | 100 | 100 | 0 | 0 | 0 | 100 | 100 | 100 | 100 |
| Casein | 200 | 200 | 200 | 200 | 200 | 200 | 200 | 200 | 200 | 200 |
| Dextrin | 132 | 132 | 132 | 132 | 132 | 132 | 132 | 132 | 132 | 132 |
| Sucrose | 100 | 100 | 100 | 100 | 100 | 100 | 100 | 100 | 100 | 100 |
| Fiber | 50 | 50 | 50 | 50 | 50 | 50 | 50 | 50 | 50 | 50 |
| Oil | 70 | 70 | 70 | 70 | 70 | 70 | 70 | 70 | 70 | 70 |
| Mineral Mix | 35 | 35 | 35 | 35 | 35 | 35 | 35 | 35 | 35 | 35 |
| Vitamin Mix | 10 | 10 | 10 | 10 | 10 | 10 | 10 | 10 | 10 | 10 |
| Cystine | 3 | 3 | 3 | 3 | 3 | 3 | 3 | 3 | 3 | 3 |
| Choline | 2.5 | 2.5 | 2.5 | 2.5 | 2.5 | 2.5 | 2.5 | 2.5 | 2.5 | 2.5 |
| Sulindac | | | | 0.2 | | | 0.2 | 0.2 | | |
| Piroxicam | | | | | 0.2 | | | | | 0.2 |
| Aspirin | | | | | | 0.2 | | | 0.2 | |

\*: Formulation of diets based on AIN93G (J. Nutr. 123: 1939-51 (1993))
Legend:
Contr: Control AIN93G diet
Syn1: Synergy1
Syn2: Synergy2
Sul: Sulindac
Piro: Piroxicam
Aspi: aspirin (acetyl salicylate)

After 1 week of acclimatisation, 100 male Fisher 344 weanling rats were divided into 10 groups of 10 rats per group and assigned the AIN93G (Control) diet or the experimental diets for 13 weeks. For the study, temperature was maintained at $21°C \pm 1°C$, relative humidity at 50%, and light and dark cycles at 12 hours each. Feed and water were provided *ad libitum.* Weekly body weights and daily feed intake were recorded.
After adaptation, all animals received two subcutaneous injections of azoxymethane (AOM) in saline at 16mg/kg body weight at 7 and 8 weeks of age. At the end of the feeding period, the animals were sacrificed using $CO_2$ euthanasia after an overnight's fast. Accordingly, the colons of the rats were removed, flushed with potassium phosphate buffer 0.1M (pH 7.2) and examined for aberrant crypt foci (ACF) and tumours.

Results

There was no difference observed in feed intake and in body weight gain between the groups.
Cecal pH was significantly ($p<0.05$) lower for the groups fed Synergy1 and Synergy2 either alone or in combination with NSAID's and the cecal weights were significantly ($p<0.05$) higher in those groups.
The effects of the diets on the AOM-induced ACF are shown in Table 2 below. The animals administered only saline (vehicle) showed no evidence of ACF formation in the colon (data not shown). In the animals fed the control diet, AOM induced an average of about 158 ACF/colon. The number of ACF induced in the rats fed the experimental diets ranged from about 35 in the group fed Synergy2 + Sulindac to about 74 in the group fed Synergy1. Fisher PLSD Test revealed that the distal segment of the colon had significantly higher ($p<0.05$) number of ACF than the proximal segment. These data are consistent with reports that the incidence of colon cancer is significantly higher in humans in the distal end rather than in the proximal colon. Feeding diets of Synergy1 and Synergy2 at 10 % levels reduced the incidence of ACF by respectively about 53% and about 68% compared to the controls. The highest ACF reduction compared to controls was seen in the group fed Synergy2 + Sulindac (about 78%) and the lowest reduction in the Sulindac group (about 53%). Reduction of total ACF followed the same pattern. The reductions were significantly (p<0.05) different from the controls.

In Table 3 below, the total number of foci containing 1, 2, 3, 4 and 5 or more aberrant crypts per focus in the proximal and the distal colon, as well as the total number of ACF, are indicated. The data show that the foci with 3, 4 and 5 or more aberrant crypts were significantly lower in rats fed experimental diets compared to rats fed control diet. It is generally accepted that the aberrant crypts with higher multiplicity have a stronger predictive value for tumour formation.

Table 2:

| Number of azoxymethane-induced aberrant crypt foci in rat colon | | | |
|---|---|---|---|
| Diet * | Number** of AOM-induced ACF in rat colon in | | |
| | proximal part | distal part | total ACF in colon |
| Control | $38.20 \pm 0.80^a$ | $119.43 \pm 1.68^a$ | $157.6 \pm 3.76^a$ |
| Syn1 | $18.24 \pm 0.46^b$ | $56.16 \pm 1.14^b$ | $74.34 \pm 4.32^b$ |
| Syn2 | $15.14 \pm 0.60^b$ | $41.01 \pm 1.08^c$ | $56.14 \pm 2.38^d$ |
| Sulindac | $11.24 \pm 0.90^b$ | $62.58 \pm 1.68^b$ | $73.87 \pm 4.25^b$ |
| Piroxicam | $12.16 \pm 1.04^b$ | $50.48 \pm 1.09^b$ | $62.64 \pm 2.86^c$ |
| Aspirin | $10.26 \pm 1.11^b$ | $54.14 \pm 1.96^b$ | $64.40 \pm 3.46^c$ |
| Sul+Syn1 | $12.42 \pm 1.04^b$ | $39.40 \pm 1.46^c$ | $51.82 \pm 4.12^e$ |
| Sul+Syn2 | $10.48 \pm 1.11^b$ | $24.16 \pm 0.92^d$ | $34.58 \pm 3.01^g$ |
| Piro+Syn1 | $11.98 \pm 1.26^b$ | $30.26 \pm 1.82^{cd}$ | $41.18 \pm 2.86^f$ |
| Aspi+Syn1 | $12.62 \pm 1.41^b$ | $34.23 \pm 1.44^c$ | $46.82 \pm 3.43^e$ |

*: the same legend applies as to Table 1

** Values are Means ± SE, n=10 for all groups.
abcefg Means within the columns with different letters are significantly different (p<0.05) by Fisher PLSD.
AOM : azoxymethane
ACF : aberrant crypt foci

Table 3:

| Number of aberrant crypt foci (ACF) in rat colon | | | | | | |
|---|---|---|---|---|---|---|
| Diet* | Number** of aberrant crypts per focus | | | | | Total ACF |
| | 1 | 2 | 3 | 4 | 5(+)*** | |
| Control | $12.22 \pm 1.18^a$ | $22.00 \pm 0.99^a$ | $40.10 \pm 3.44^a$ | $52.04 \pm 4.20^a$ | $31.24 \pm 2.46^a$ | $157.6 \pm 3.76^a$ |
| Syn1 | $4.81 \pm 0.46^c$ | $18.24 \pm 1.14^a$ | $22.84 \pm 1.86^b$ | $13.41 \pm 2.08^c$ | $15.04 \pm 0.99^b$ | $74.34 \pm 4.32^b$ |
| Syn2 | $3.52 \pm 0.09^c$ | $15.32 \pm 1.86^{ab}$ | $18.14 \pm 1.42^b$ | $9.02 \pm 1.46^d$ | $10.14 \pm 1.02^c$ | $56.14 \pm 2.38^d$ |
| Sulindac | $4.08 \pm 0.48^c$ | $12.14 \pm 2.04^b$ | $20.37 \pm 2.40^b$ | $20.48 \pm 2.48^b$ | $16.80 \pm 0.78^b$ | $73.87 \pm 4.25^b$ |
| Piro | $4.18 \pm 1.04^c$ | $13.16 \pm 1.54^b$ | $14.68 \pm 1.89^c$ | $14.78 \pm 1.94^c$ | $14.24 \pm 0.94^b$ | $62.64 \pm 2.86^c$ |

*: the same legend applies as to Table 1

**: values are Means ± SE, n=10 for all groups.
abcdefg Means within the columns with different letters are significantly different (p<0.05) by Fisher PLSD.

***: 5(+) = 5 and more aberrant crypts per focus.

Table 3: (continued)

| Diet* | Number** of aberrant crypts per focus | | | | | Total ACF |
|---|---|---|---|---|---|---|
| | 1 | 2 | 3 | 4 | 5(+)*** | |
| Aspi | 6.50 ± 1.12[b] | 12.48 ± 1.23[b] | 17.42 ± 1.94[b] | 15.86 ± 1.82[c] | 12.14 ± 0.86[b] | 64.40 ± 3.46[c] |
| Sul +Syn1 | 4.33 ± 0.40[c] | 16.19 ± 1.46[b] | 12.20 ± 1.26[c] | 8.62 ± 1.62[d] | 10.48 ± 0.92[c] | 51.82± 4.12[e] |
| Sul +Syn2 | 4.01 ± 0.48[c] | 7.62 ± 0.94[c] | 10.92 ± 1.42[c] | 5.84 ± 0.98[d] | 6.20 ± 0.52[c] | 34.58 ± 3.01[g] |
| Piro +Syn1 | 4.26 ± 0.80[c] | 9.26 ± 0.98[c] | 10.42 ± 1.64[c] | 6.98 ± 0.95[d] | 10.26 ± 0.19[c] | 41.18 ± 2.86[f] |
| Aspi +Syn1 | 41.18± 0.91[c] | 12.00 ± 1.42[b] | 12.16 ± 1.89[c] | 7.20 ± 0.12[d] | 11.28± 0.84[b] | 46.82± 3.43[e] |

\*: the same legend applies as to Table 1

\*\*: values are Means ± SE, n=10 for all groups.
abcdefg Means within the columns with different letters are significantly different ($p<0.05$) by Fisher PLSD.

\*\*\*: 5(+) = 5 and more aberrant crypts per focus.

Example 2

In example 2, experiments similar to the ones of example 1 were carried out, to examine the anti-cancer effects of a composition containing a combination of RAFTILINE® HP and sulindac. The composition of the diets is shown in Table 4 below. The 4 groups each of 10 Fisher 344 male rats were given a diet containing either control diet, control diet + RAFTILINE®HP, control diet + sulindac, or control diet + RAFTILINE®HP + sulindac (RAFTILINE® : trade name of RT/TS, Belgium). The protocol of the experiments was the same as the one of example 1.

The experiments of example 2 gave the following results.
Body weight gains of the rats were not different in the rats fed inulin at the 10% level compared to the groups fed the (AIN 93G) control diet and the sulindac. The differences in mean daily feed intake were not statistically significant for the rats fed the control and the experimental diets.
Cecal pH was lower in the groups being fed the inulin-containing diets compared to the control diet ($p<0.05$), whereas cecal weights were higher in the inulin groups than in the control group.
The effects of the diets on the AOM-induced ACF are shown in Tables 5 and 6 below.
The rats receiving saline (vehicle) injection and fed the control diet showed no evidence of ACF formation in the colon (data not shown).
The results of the study demonstrated that the rats fed a diet containing a combination of sulindac and RAFTILINE®HP according to the invention had the highest reduction of AOM-induced colonic ACF compared to the control group. AOM treatments induced an average of about 155 ACF/colon in the rats fed the control diet. ACF were predominantly observed in the distal colon (about 115). In the experiment, the highest reduction of AOM-induced colonic ACF compared to the control group was seen in Fisher 344 male rats fed a diet containing sulindac + RAFTILINE®HP. In this group, compared to the control diet group, a reduction of ACF of about 63% in the proximal colon and of about 65% in the distal colon and an overall reduction in ACF of about 66 % was observed.
Administration of sulindac in the diet showed, compared to the control diet, a reduction of ACF in the proximal and distal colon of respectively about 74% and about 44% and an overall reduction of about 52%. Feeding RAFTILINE®HP in the diet showed about 67% reduction in the ACF in the proximal colon, about 39% reduction in the ACF in the distal colon, and a total of about 46% reduction in ACF in the total colon, compared to the control group. Feeding sulindac + RAFTILINE®HP in the diet reduced total ACF by about 65% as compared to the control diet.
Consumption of diets containing RAFTILINE®HP, sulindac and a combination of RAFTILINE®HP + sulindac showed a reduction of total colonic ACF by about 46%, 52% and 66% respectively compared to the control diet.

Examination of the rat colons showed that the distal end had significantly ($p<0.05$) higher number of aberrant crypts than the proximal end when compared using Tukey's Studentised range test. The total number of foci containing 1, 2, 3, 4 and 5 or more aberrant crypts were counted in the distal and proximal regions. The results are given in Table 6 and indicate that foci with 3, 4 and 5 or more aberrant crypts were significantly lower ($p<0.05$) in rats fed Sulindac and in rats fed a combination of Sulindac and RAFTILINE®HP of the invention, compared to the other groups.

Table 4:

| Composition of the diets | | | | |
|---|---|---|---|---|
| Diet (in g/kg) | Control | RAFTI- HP * | Sulindac | Sulindac + RAFTI- HP* |
| Corn Starch | 397.5 | 297.5 | 397.5 | 297.5 |
| Inulin | 0 | 100.00 | 0 | 100.00 |
| Casein (85% protein) | 200.00 | 200.00 | 200.00 | 200.00 |
| Dextrinised corn starch (90-94% tetrasaccharides) | 132.00 | 132.00 | 132.00 | 132.00 |
| Sucrose | 100.00 | 100.00 | 100.00 | 100.00 |
| Soybean oil (no additive) | 70.00 | 70.00 | 70.00 | 70.00 |
| Fiber[1] | 50.00 | 50.00 | 50.00 | 50.00 |
| Mineral Mix (AIN 93G-MX) | 35.00 | 35.00 | 35.00 | 35.00 |
| Vitamin Mix (AIN-93 VX) | 10.00 | 10.00 | 10.00 | 10.00 |
| L-Cystine | 3.00 | 3.00 | 3.00 | 3.00 |
| Choline bitartrate (41.1 % Choline) | 2.50 | 2.50 | 2.50 | 2.50 |
| Sulindac | 0 | 0 | 0.2 | 0.2 |

\* RAFTI-HP = RAFTILINE®HP (RAFTILINE®: trade name of RT/TS, Belgium)

Fiber[1] : Cellulose Solk-Floc R 200 FCC (FS&D, St. Louis, MO, USA).

Table 5:

| Number of azoxymethane-induced aberrant crypt foci in rat colon | | | |
|---|---|---|---|
| Diet * (n=number of rats) | Number ** of AOM-induced ACF in rat colon in | | |
| | proximal part | distal part | Total ACF in colon |
| Control (n =12) | $39.92 \pm 0.82^a$ | $114.17 \pm 1.57^a$ | $155.42 \pm 1.64^a$ |
| RAFTILINE®HP (n =10) | $13.30 \pm 1.45^c$ | $70.20 \pm 1.18^c$ | $83.50 \pm 1.67^c$ |
| Sulindac (n =10) | $10.58 \pm 0.90^c$ | $64.50 \pm 1.54^d$ | $75.08 \pm 1.93^d$ |
| Sulindac + RAFTILINE®HP (n =12) | $14.80 \pm 0.93^c$ | $40.40 \pm 2.56^e$ | $53.20 \pm 1.29^e$ |

\* : the same legend applies as to Table 1

\*\* : Values are Means ± SE, n= as indicated.

[acde] Means within the columns with different letters are significantly different ($p < 0.05$) by Fisher PLSD.

AOM : azoxymethane ACF : aberrant crypt foci

Table 6:

| Diet (n: number of rats) | Number * of aberrant crypts per focus | | | | | Total ACF |
|---|---|---|---|---|---|---|
| | 1 | 2 | 3 | 4 | 5(+)** | |
| Control (n=12) | 9.08 ± 1.04 [a] | 19.17 ± 0.91 [a] | 41.75 ± 3.48[a] | 50.08 ± 3.22 [a] | 30.75 ± 1.46[a] | 155.42 ± 1.64[a] |
| RAFTILINE®HP (n =10) | 3.10 ± 0.43[b] | 11.20 ± 0.98[b] | 25.90 ± 1.20 [c] | 25.80 ± 0.65 [b] | 17.40 ± 0.67[c] | 83.50 ± 1.67 [c] |
| Sulindac (n=10) | 3.50 ± 0.38 [b] | 11.67 ± 1.19[b] | 22.17± 1.03 [c] | 22.50 ± 0.86[b] | 15.33 ± 0.45 [c] | 75.08 ± 1.93[d] |
| Sulindac + RAFTILINE®HP (n =12) | 2.40 ± 0.31[b] | 5.00 ± 0.39 [c] | 15.00 ± 0.54 [d] | 15.60 ± 0.50 [c] | 12.50 ± 0.54 [c] | 53.20 ± 1.20 [e] |

\* : values are Means ± SEM, n = as indicated.

[abcde] Means within the column with different letters are significantly different (p<0.05) by Turkey's Studentised Range Test.

\*\* : 5(+) = 5 and more aberrant crypts per focus.

<u>Conclusion</u>

From the comparative data presented in Tables 2, 3 and 5, it clearly follows that the combination of a FDF, in particular inulin, and a non-steroidal anti-inflammatory drug (NSAID) presents a synergistic therapeutic effect on cancer/ a synergistic anti-cancer effect in rats.

As a result thereof, the compositions of the invention present considerable advantages over conventional anti-cancer compositions. For example, compared to a conventional composition containing the same concentration of anti-cancer drug, the therapeutic effect of a composition of the invention is significantly increased. This increase may lead to a considerable improvement of control of the cancer disease by better preventing and inhibiting carcinogenesis and/or recurrence of cancer, by improving the curative effects vis-à-vis cancer, by further reducing the risk of recurrence of cancer, or even by restoring to a larger extent the normal physiological functions and body structures affected by cancer.

On the other hand, whereas a conventionally therapeutic dose of a NSAID for use as anti-cancer drug may be too toxic for the patient or may provoke too pronounced undesirable side effects, the synergistic effects of the combination of the invention enable to use NSAID's as anti-cancer drug at a lower dose than said conventionally therapeutic dose, while maintaining the same desirable level of therapeutic effects, and without provoking a too high toxicity or too pronounced undesirable side effects.

In addition, in view of the presence of FDF in the compositions of the invention, humans and animals treated with a pharmaceutical composition of the invention will also enjoy the known beneficial effects resulting from the intake of fermentable dietary fibers, such as prebiotic effects.

Consequently, it can be concluded that the combination according to the invention and a pharmaceutical composition comprising said combination provide a considerable improvement for the prevention and inhibition of carcinogenesis and recurrence of cancer and for the treatment of cancer in humans and in animals.

<u>References</u>

Giardiello F. et al., New England Journal of Medicine, Vol. 328 (18),1313-1316, (1993)
Howe, G.R. et al., J. Natl. Cancer Inst., Vol. 84,1887-1896, (1992)
Leibovici J. et al., Br. J exp.Path. Vol 64, 239-244, (1983)
De Leenheer, L., Carbohydrates as Organic Raw Materials, Vol. III, 67-92, (1996)

**Claims**

1. Pharmaceutical composition with anti-cancer properties, **characterised in that** it comprises a combination consisting of an effective amount of a fermentable dietary fiber (herein FDF) and an effective amount of a non-steroidal anti-inflammatory drug (herein NSAID) with cyclo-oxygenase2 (herein COX2) inhibiting activity, in which combination the FDF is present in the weight ratio FDF : NSAID of minimum 1:1.

2. Pharmaceutical composition according to claim 1, wherein in the combination of FDF and NSAID the FDF component is present in the weight ratio FDF : NSAID ranging from 6000 :1 to 1 : 1.

3. Pharmaceutical composition according to any one of claims 1 to 2, wherein the NSAID is selected from the group consisting of a non-selective cyclo-oxygenase (herein COX) inhibitor and a selective cyclo-oxygenase2 (herein COX2) inhibitor.

4. Pharmaceutical composition according to claim 3, wherein the NSAID is selected from the group consisting of sulindac, sulindac sulfide, sulindac sulfoxide, sulindac sulfone, aspirin, piroxicam, ketyoprofen, diclofenac, ibuprofen, indomethacin, celecoxib, nimesulide, omeprazole, and mofarotene.

5. Pharmaceutical composition according to any one of claims 1 to 4, wherein the fermentable dietary fiber is selected from the group consisting of resistant starch, fructan, pectin, galactomannan, gums, and partial hydrolysates of said compounds, beta-glucan, and FDF of the class of oligosaccharides.

6. Pharmaceutical composition according to claim 5, wherein the fructan is inulin, oligofructose or a mixture thereof.

7. Pharmaceutical composition according to claim 5 wherein the fructan is chicory inulin with an av. DP of at least 23.

8. Pharmaceutical composition according to claim 5, wherein the fructan is a mixture of an easily fermentable inulin (EFI) and a hardly fermentable inulin (HFI) in a weight ratio EFI : HFI ranging from 10 : 90 to 70 : 30 and wherein the total content of inulin with DP 9 and DP 10 is maximally 5 % by weight calculated on the total weight of EFI and HFI.

9. Pharmaceutical composition according to claim 8, wherein the easily fermentable inulin (EFI) is oligofructose with a DP ranging from 2 to 9 and the hardly fermentable inulin (HFI) is chicory inulin with an av. DP of 23 to 25, with a total content of inulin with a DP 9 and DP 10 of maximally 5 % by weight, in a weight ratio EFI : HFI of 1 to 1 or 1 to 2.

10. Pharmaceutical composition according to any one of claims 1 to 9, wherein the FDF component and the NSAID component of the FDF/NSAID combination are present in the same galenic formulation.

11. Pharmaceutical composition according to any one of claims 1 to 9, wherein the FDF component and the NSAID component of the FDF/NSAID combination are present in separate galenic formulations which together form the pharmaceutical composition.

12. Pharmaceutical composition according to claim 10 or 11, wherein the galenic formulation or the separate galenic formulations forming the pharmaceutical composition are in a galenic form suitable for oral administration or for tube feeding.

13. Pharmaceutical composition according to claim 12 wherein the FDF component of the combination of the pharmaceutical composition is present in functional food or feed.

14. Pharmaceutical composition according to claims 10 or 11, wherein the NSAID is present in a galenic formulation that is suitable for oral or for parenteral administration.

15. Pharmaceutical composition according to any one of claims 1 to 14 for use as a medicament for the prevention or inhibition of carcinogenesis and recurrence of cancer or for treatment of cancer in humans or vertebrate animals.

16. Use of a combination of a FDF and a NSAID defined in any one of claims 1 to 9 for the manufacture of a pharmaceutical composition according to any one of claims 1 to 14 for the prevention or inhibition of carcinogenesis and recurrence of cancer or for the treatment of cancer in humans or vertebrate animals.

17. Method for the prevention or inhibition of carcinogenesis and the recurrence of cancer or for the treatment of cancer in a humans or vertebrate animals comprising administering to said being in need of such treatment a therapeutically effective dose of a pharmaceutical composition defined in any one of claims 1 to 14.

18. Method according to claim 17 wherein the FDF component and the NSAID component of the FDF/NSAID combination of the pharmaceutical composition are present in the same galenic formulation.

19. Method according to claim 17 wherein the FDF component and the NSAID component of the FDF/NSAID combination of the pharmaceutical composition are present in separate galenic formulations constituting together the pharmaceutical composition.

**European Patent**

**Office**

## PARTIAL EUROPEAN SEARCH REPORT

which under Rule 45 of the European Patent Convention
shall be considered, for the purposes of subsequent
proceedings, as the European search report

**Application Number**

EP 02 02 1644

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 00 28974 A (DEXXON LTD ;FLASHNER MOSHE (IL); LERNER ITZHAK E (IL); PENHASI ADE) 25 May 2000 (2000-05-25) <br> * abstract * <br> * page 1, line 7 - line 8 * <br> * page 9, line 5 - line 12 * <br> * page 10, line 19 - line 29 * <br> * page 11, line 8 - line 11 * <br> * page 15, line 24 - page 16, line 15 * <br> * page 21, line 17 - line 31 * <br> * page 29, line 12 - line 27 * <br> * page 33, line 18 - line 21 * <br> * page 45, line 4 - line 6 * <br> * claims 1-115 * <br> --- | 4-19 | A61K31/19 A61K31/715 A61P35/00 //(A61K31/19, 31:715) |
| D,A | WO 99 59600 A (ROBERFROID MARCEL ;TIENSE SUIKERRAFFINADERIJ N V (BE); FRIPPIAT AN) 25 November 1999 (1999-11-25) <br> * abstract * <br> * page 1, line 6 - line 9 * <br> * page 4, line 3 - page 5, line 15 * <br> * tables 1,2 * <br> * claims 1-20 * <br> --- <br> -/-- | 4-19 | |

**TECHNICAL FIELDS SEARCHED (Int.Cl.7)**

A61K

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| MUNICH | 30 January 2003 | Taylor, G.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

| | European Patent Office | INCOMPLETE SEARCH SHEET C | Application Number EP 02 02 1644 |
|---|---|---|---|

Although claims 17-19 are directed to a method of treatment of the human/animal body (Article 52(4) EPC), the search has been carried out and based on the alleged effects of the compound/composition.

--------------------

Claim(s) searched incompletely:
    4-7, 9-19

Claim(s) not searched:
    1-3, 8

Reason for the limitation of the search:

Present claims 1-3 and 8 relate to a composition defined by reference to a desirable characteristic or property, namely

a fermentable dietary fibre;
a non-steroidal anti-inflammatory drug with cyclo-oxygenase2 inhibiting activity;
an easily fermentable inulin;
a hardly fermentable inulin.

The claims cover all compositions having this characteristic or property, whereas the application provides support within the meaning of Art. 84 EPC and/or disclosure within the meaning of Art. 83 EPC for only a very limited number of such compositions. In the present case, the claims so lack support, and the application so lacks disclosure, that a meaningful search over the whole of the claimed scope is impossible.

Independent of the above reasoning, the claims also lack clarity (Art. 84 EPC). An attempt is made to define the compositions by reference to a result to be achieved. Again, this lack of clarity in the present case is such as to render a meaningful search over the whole of the claimed scope impossible. Consequently, the search has been carried out for those parts of the claims which appear to be clear, supported and disclosed, namely those parts relating to the compositions as defined by claims 4 and 5 in combination. The remaining dependent claims have been searched as if depndent upon the combination of claims 4 and 5 as the first independent claim.

European Patent
Office

**PARTIAL EUROPEAN SEARCH REPORT**

Application Number

EP 02 02 1644

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| D,A | EP 0 692 252 A (RAFFINERIE TIRLEMONTOISE SA) 17 January 1996 (1996-01-17) <br> * abstract * <br> * page 2, line 49 - page 3, line 26 * <br> * claims 1-12 * <br> --- | 4-19 | |
| D,A | WO 98 52578 A (TIENSE SUIKERRAFFINADERIJ N V ;FRIPPIAT ANNE (BE); LOO JAN VAN (BE) 26 November 1998 (1998-11-26) <br> * abstract * <br> * page 1, line 8 - line 14 * <br> * page 5, line 28 - page 7, line 8 * <br> * claims 1-22 * <br> --- | 4-19 | |
| D,A | WO 01 60176 A (FRIPPIAT ANNE ;LOO JAN VAN (BE); SMITS GEORGES (BE); TIENSE SUIKKE) 23 August 2001 (2001-08-23) <br> * abstract * <br> * page 1, line 4 - line 11 * <br> * claims 1-29 * <br> --- | 4-19 | **TECHNICAL FIELDS SEARCHED** (Int.Cl.7) |
| A | CORPET D E ET AL: "CARRAGEENAN GIVEN AS A JELLY, DOES NOT INITIATE, BUT PROMOTES THE GROWTH OF ABERRANT CRYPT FOCI IN THE RAT COLON" <br> CANCER LETTERS, NEW YORK, NY, US, <br> vol. 114, 19 March 1997 (1997-03-19), <br> pages 53-55, XP000986974 <br> ISSN: 0304-3835 <br> * the whole document * <br> ----- | 4-19 | |

**ANNEX TO THE EUROPEAN SEARCH REPORT
ON EUROPEAN PATENT APPLICATION NO.**                    EP 02 02 1644

This annex lists the patent family members relating to the patent documents cited in the above-mentioned European search report.
The members are as contained in the European Patent Office EDP file on
The European Patent Office is in no way liable for these particulars which are merely given for the purpose of information.

30-01-2003

| Patent document cited in search report | | Publication date | Patent family member(s) | | Publication date |
|---|---|---|---|---|---|
| WO 0028974 | A | 25-05-2000 | US | 6231888 B1 | 15-05-2001 |
| | | | AU | 1175000 A | 05-06-2000 |
| | | | EP | 1131058 A1 | 12-09-2001 |
| | | | WO | 0028974 A1 | 25-05-2000 |
| WO 9959600 | A | 25-11-1999 | EP | 0958825 A1 | 24-11-1999 |
| | | | AT | 225179 T | 15-10-2002 |
| | | | DE | 69903283 D1 | 07-11-2002 |
| | | | DK | 1079840 T3 | 04-11-2002 |
| | | | WO | 9959600 A1 | 25-11-1999 |
| | | | EP | 1079840 A1 | 07-03-2001 |
| EP 0692252 | A | 17-01-1996 | DE | 69520528 D1 | 10-05-2001 |
| | | | DE | 69520528 T2 | 27-09-2001 |
| | | | EP | 0692252 A1 | 17-01-1996 |
| | | | US | 5721345 A | 24-02-1998 |
| WO 9852578 | A | 26-11-1998 | EP | 0879600 A1 | 25-11-1998 |
| | | | AU | 7654898 A | 11-12-1998 |
| | | | CN | 1255855 T | 07-06-2000 |
| | | | WO | 9852578 A1 | 26-11-1998 |
| | | | EP | 0983072 A1 | 08-03-2000 |
| | | | JP | 2001526671 T | 18-12-2001 |
| | | | US | 2002177561 A1 | 28-11-2002 |
| WO 0160176 | A | 23-08-2001 | EP | 1125507 A1 | 22-08-2001 |
| | | | AU | 4239201 A | 27-08-2001 |
| | | | WO | 0160176 A1 | 23-08-2001 |
| | | | EP | 1259125 A1 | 27-11-2002 |

EPO FORM P0459

For more details about this annex : see Official Journal of the European Patent Office, No. 12/82